Europäisches Patentamt

European Patent Office

Office européen des brevets

(11) Veröffentlichungsnummer: **0 154 922 B1**

# EUROPÄISCHE PATENTSCHRIFT

(45) Veröffentlichungstag der Patentschrift: **13.03.91**

(21) Anmeldenummer: **85102442.2**

(22) Anmeldetag: **05.03.85**

(51) Int. Cl.⁵: **C08L 83/07, C08K 5/00**

Die Akte enthält technische Angaben, die nach
dem Eingang der Anmeldung eingereicht wurden
und die nicht in dieser Patentschrift enthalten sind.

(54) Strukturviskos eingestellte Silikonpasten und deren Verwendung als Abformmassen.

(30) Priorität: **16.03.84 DE 3409720**

(43) Veröffentlichungstag der Anmeldung:
**18.09.85 Patentblatt 85/38**

(45) Bekanntmachung des Hinweises auf die
Patenterteilung:
**13.03.91 Patentblatt 91/11**

(84) Benannte Vertragsstaaten:
**AT BE CH DE FR GB IT LI LU NL SE**

(56) Entgegenhaltungen:
**EP-A- 16 988       EP-A- 0 046 907
BE-A- 877 987       DE-A- 2 623 960
DE-A- 2 736 421     US-A- 4 035 453**

(73) Patentinhaber: **BAYER AG**

**W-5090 Leverkusen 1 Bayerwerk(DE)**

(72) Erfinder: **Voigt, Reiner, Dipl.-Ing.
Gustav-Radbruch-Strasse 14
W-5090 Leverkusen 3(DE)**
Erfinder: **Schwabe, Peter, Dr.
Dudweiler Strasse 17
W-5090 Leverkusen 1(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft mittels hydriertem Rizinusöl strukturviskos eingestellte Silikonpasten, die sich zur Herstellung genauer Abformungen von Zähnen und Schleimhäuten eignen. Bei den erfindungsgemäßen Pasten handelt es sich um kaltvulkanisierende Zweikomponenten-Silikonkautschuksysteme aus einer "Basispaste" und einer "Katalysatorpaste", die vor der Anwendung miteinander vermischt werden und dann nach ca. 2 bis 5 Minuten bei Raumtemperatur vernetzen.

Silikonpasten zur Zahnabformung sind weit verbreitet. Im allgemeinen bestehen sie aus einem mit Füllstoffen vermischten Silikonöl auf Basis eines hydroxylendgestoppten Polydimethylsiloxans, welches je nach Applikationsmethode in verschiedenen Konsistenzen erhältlich ist, und einer flüssigen oder pastenförmigen Härterkomponente, die ein Metallsalz einer Monocarbonsäure als Katalysator und einen Kieselsäureester als Vernetzer enthält. Vor der Anwendung werden die beiden Komponenten gemischt und vernetzen dann, auf Abformlöffel appliziert, im Mund des Patienten innerhalb von ca. 2 bis 5 Minuten infolge einer Polykondensationsreaktion zu einem Polymeren mit gummiartiger Konsistenz.

Seit einigen Jahren sind Abformmassen auf Basis von Vinylsilikonen bekannt, die nach einer Polyadditionsreaktion vernetzen. Diese Massen bestehen aus zwei Pasten, einer Silikonöl, Füllstoff und Vernetzer enthaltenden Basispaste und einer Katalysatorpaste aus Silikonöl, Füllstoffen und Katalysator.

Bei dem Silikonöl handelt es sich um ein vinylendgestopptes Polydimethylsiloxan, der Vernetzer enthält Polydimethylsiloxane mit reaktiven SiH-Gruppen und der Katalysator besteht aus einem Platinkomplex. Additionsvernetzende Systeme haben neben der größeren Dimensionsgenauigkeit der Abformung noch den Vorteil einer besseren Dosierbarkeit von Basis- und Katalysatorpaste infolge gleicher Pastenviskosität und des abgestimmten Mischungsverhältnisses von 1:1 der beiden Pasten sowie einer Geschmacks- und Geruchlosigkeit der Pasten.

In der Zahnheilkunde werden Abformungen nach verschiedenen Methoden, wie z.B. Doppelmisch-, Doppelabform- und Einphasentechnik hergestellt, die Pasten in verschiedenen Viskositätsklassen, wie z.B. knetbar, hoch-, mittel- und niedrigviskos, voraussetzen. Einfluß auf die Pastenviskosität haben die Silikonölviskosität, das mengenmäßige Verhältnis Silikonöl : Füllstoff, die Affinität des Füllstoffes zum Silikonöl ("Ölzahl"), die Dichte und die spez. Oberfläche des Füllstoffes.

Bei den kondensationsvernetzenden Abformmassen auf Basis hydroxylendgestoppter Polydimethylsiloxane werden in der Regel Calciumsilikat, Calciumcarbonat, Calciumsulfat, Talkum, Bimsstein-, Quarz- oder Cristobalitmehle als Füllstoffe eingesetzt. Um eine Sedimentation der Füllstoffe in den Pasten zu verhindern, werden hochdisperse Kieselsäuren, Emulgatoren wie ethoxylierter Fettalkohol (US-PS 3 082 527), Rizinusöl oder Paraffin/Vaseline (FR-A 2 366 334) zugesetzt.

Das Spektrum der Füllstoffe, die in additionsvernetzenden Abformmassen auf Basis vinylendgestoppter Polydimethylsiloxane eingesetzt werden können, ist wesentlich kleiner. So können Füllstoffe Spuren von Schwefel, Eisen, Nickel oder Zinn in die Paste bringen und damit den Platin-Katalysator desaktivieren. Andererseits reagieren Feuchtigkeit und/oder SiOH-Gruppen auf der Füllstoffoberfläche mit den SiH-Gruppen des Vernetzers unter Wasserstoffbildung, besonders wenn wäßrige Füllstoffaufschlämmungen eine Basizität oder Acidität aufweisen. Im allgemeinen werden in diese Pasten Calciumcarbonat, Quarz- und Cristobalitmehle, deren Oberflächen vorzugsweise mit Substanzen auf Basis Silazan oder andern Oberflächenbelackungen auf Silikonbasis beladen sind, als Füllstoffe eingesetzt. Die Sedimentationsneigung verringert man mit hochdispersen Kieselsäuren, deren Oberflächen ebenfalls beladen sein sollten.

Mit Hilfe eines Zusatzes von hochdispersen Kieselsäuren ließen sich bei Silikonabformmassen strukturviskose und/oder thixotrope Eigenschaften erzielen. Dennoch wurde dieser Effekt nie bewußt genutzt, um additionsvernetzende Massen zu erhalten, die nach dem Vermischen von Basis- und Katalysatorpaste, beim Auftragen auf den Vorabdruck bzw. beim Ausdrücken aus der Spritze eine niedrige Viskosität besitzen und im Mund des Patienten infolge der Strukturviskosität einen Viskositätsanstieg aufzeigen, der das Abtropfen der Masse von den Zähnen und/oder das Fließen der Masse in den Rachenraum verhindert. Der Grund dafür liegt darin, daß bei den hochdispersen Kieselsäuren einerseits die Bildung der Thixotropie und/oder Strukturviskosität in den Pasten durch SiOH-Gruppen auf der Kieselsäure-Oberfläche bewirkt wird, andererseits jedoch wie oben beschrieben, die SiOH-Gruppen durch Behandlung mit Silazanen vor Verwendung der hochdispersen Kieselsäuren in den Vinylsilikon-Abformmassen blockiert werden sollten. Hochdisperse Kieselsäuren fallen daher als Strukturbildner für derartige Massen aus. Es wurde nun gefunden, daß hydriertes Rizinusöl in additionsvernetzenden Silikon-Abformmassen eine hervorragende Strukturviskosität aufbaut, ohne die Lagerstabilität der Basis- und Katalysatorpaste der Vinylsilikon-Abformmassen negativ zu beeinflussen.

Aus EP-A 0 016 988 ist bekannt, daß bestimmte oberflächenaktive Substanzen als Thixotropierhilfsmittel für kondensationsvernetzende Silikonmassen verwendet werden können. Eine Übertragung dieser

2

technischen Lehre auf additionsvernetzende Systeme ist jedoch nicht ohne weiteres möglich: So führen z.B. die gemäß EP-A 0 016 988 bevorzugten Thixotropierhilfsmittel nämlich Glycerin-mono-distearat-Polyglykolether, zu einer Beeinträchtigung der Katalysatoraktivität in additionsvernetzenden Pasten und kommen daher für den praktischen Einsatz in solchen Systemen nicht in Frage.

Gegenstand der Erfindung sind additionsvernetzende Massen auf Polysiloxanbasis, enthaltend

a) ein Organosiloxan mit zwei oder mehr Vinylgruppen im Molekül,

b) ein Organohydrogenpolysiloxan als Vernetzer,

c) einen Katalysator zur Beschleunigung der Additionsreaktion,

d) hydrophobe Füllstoffe, sowie gegebenenfalls

e) Farbstoffe welche dadurch gekennzeichnet sind, daß sie zusätzlich hydriertes Rizinusöl mit einem Schmelzbereich von 80 bis 95 °C als Strukturbildner enthalten.

Gegenstand der Erfindung ist auch die Verwendung der erfindungsgemäß thixotropierten strukturviskosen Pasten als Abformmassen, insbesondere für Zahn- und Schleimhautabformungen.

Die erfindungsgemäßen Pasten enthalten vorzugsweise 0,1 bis 3 Gew.-%, besonders bevorzugt 0,2 bis 1,8 Gew.-%, bezogen auf Gesamtpaste an hydriertem Rizinusöl.

Ein Hauptvorteil der erfindungsgemäßen Vinylsilikon-Pasten ist ihre Strukturviskosität. Beim Ausdrücken der Pasten aus der Tube erhält man infolge ihrer hohen Viskosität standfeste Stränge, die ein genaues Dosieren von Basis- und Katalysatorpaste ermöglichen. Das Mischen der beiden Pasten wird andererseits durch Viskositätsabbau infolge Scherbeanspruchung erleichtert. Nach Beendigung des Mischvorganges ist die Masse jedoch umgehend wieder höherviskos, so daß sie nach Applikation auf den Abform-Löffel nicht von diesem ablaufen kann. Beim Einbringen des gefüllten Abformlöffels in den Mund wird die Masse im Bereich der abzuformenden Partien (Zähne, Schleimhaut, Kieferwall) wieder niedrigviskos und zeichnet so die Mundsituation gut ab; andererseits tropft die Masse nicht in den Rachenraum des Patienten, was zur Auslösung eines Würgereizes und zur Unbrauchbarkeit der Abformung führen kann. Bei der Applikation mittels einer Spritze wird das Gemisch von Basis- und Katalysatorpaste in niedrigviskoser Konsistenz (infolge der Scherbeanspruchung in der Spritzendüse) z.B. um abzuformende Zahnstümpfe gelegt. Infolge eines raschen Viskositätsaufbaus fließt die Masse nicht vom Applikationsort ab und tropft auch nicht in den Rachenraum. Andererseits fließt die Masse mit der auf dem unmittelbar darauf eingeführten Abformlöffel befindlichen Masse zusammen. Eine genaue Abformung der Mundsituation und (nach dem Ausgießen der Abformung mit einer wäßrigen Gipsaufschlämmung und deren Aushärtung) eine detailgetreue Wiedergabe des Gipsmodells sind das Ergebnis der erfindungsgemäßen Vinylsilikon-Abformmassen.

Bestandteile der erfindungsgemäßen Vinylsilikon-Pasten sind Silikonöl (a), Vernetzer (b), Katalysator (c), Füllstoffe (d), Farbstoffe (e) und schließlich organische Strukturbildner (f).

Bei dem Silikonöl (a) handelt es sich um an sich bekannte vinylendgestoppte Polydimethylsiloxane, deren Viskosität im Bereich von 500 bis 100.000 mPa.s bei 20 °C liegen kann, je nach gewünschter Konsistenz der formulierten Paste.

Der Vernetzer (b) ist ein ebenfalls an sich bekanntes Polydimethylsiloxan, welches in seinem Molekül Wasserstoffatome an mindestens zwei Siliciumatomen aufweist.

Bei dem Katalysator (c) handelt es sich z.B. um einen Platin-Komplex, der aus Hexachlorplatin-IV-säure hergestellt wurde. Auch diese Verbindungen sind an sich bekannt.

Unter den Füllstoffen (d) versteht man z.B. Calciumcarbonat, Quarz- und Cristobalitmehle, sowie gefällte und pyrogen hergestellte Siliciumdioxide, deren Oberflächen vorzugsweise mit Substanzen auf Basis von Silazan oder anderen reaktiven funktionellen Silikonverbindungen beladen wurden.

Farbstoffe (e) werden zur Unterscheidung der Basis und Katalysatorpaste und zur Mischkontrolle eingesetzt. Anorganische und organische Farbpigmente werden bevorzugt.

Der erfindungsgemäß einzusetzende organische Strukturbildner ist ein hydriertes Rizinusöl, dessen Schmelzbereich bei 80 bis 95 °C, vorzugsweise bei 82 bis 90 °C liegt.

Die nachfolgenden Beispiele, in denen alle Teile Gewichtsteile bedeuten, erläutern die Erfindung.

Beispiel 1 (Vergleichsversuch)

Die Basispaste wurde hergestellt durch Vermischen in einem Kneter von 340 Teilen vinylendgestopptem Polydimethylsiloxan mit einer Viskosität von 10.000 mPa.s bei 20 °C, 210 Teilen dimethylhydrogensilylendgestopptem Polydimethylsiloxan mit einer Viskosität von 120 mPa.s bei 20 °C, 430 Teilen silanisiertem Quarzfeinstmehl und 20 Teilen anorganischem Farbpigment.

Die Herstellung der Katalysatorpaste erfolgte in einem Kneter durch Vermischen von 548 Teilen vinylendgestopptem Polydimethylsiloxan mit einer Viskosität von 5.000 mPa.s bei 20 °C, 450 Teilen des obengenannten Quarzfeinstmehl, 1,8 Teilen Titandioxid und 0,2 Teilen eines Komplexes aus Platin und Divinyltetramethyldisiloxan.

Beispiel 2 (Vergleichsversuch)

In einem Kneter wurde die Basispaste hergestellt durch Vermischen von 340 Teilen vinylendgestopptem Polydimethylsiloxan mit einer Viskosität von 10.000 mPa.s bei 20° C, 210 Teilen dimethylhydrogensilyl-endgestopptem Polydimethylsiloxan mit einer Viskosität von 120 mPa.s bei 20° C, 380 Teilen des Quarzfeinstmehls aus Beispiel 1, 50 Teilen gefällter und oberflächlich mit Silanen beladener Kieselsäure mit einer spez. Oberfläche von 90 m²/g nach BET und 20 Teilen anorganischem Farbpigment.

Die Katalysatorpaste wurde in einem Kneter hergestellt durch Vermischen von 548 Teilen vinylendgestopptem Polydimethylsiloxan mit einer Viskosität von 5000 mPa.s bei 20° C, 400 Teilen des Quarzfeinstmehls von Beispiel 1, 50 Teilen der obengenannten gefällten Kieselsäure, 1,8 Teilen Titandioxid und 0,2 Teilen des Platin-siloxan-Komplexes nach Beispiel 1.

Beispiel 3 (erfindungsgemäß)

Die Basispaste wurde hergestellt durch Vermischen in einem Kneter von 330 Teilen vinylendgestopptem Polydimethylsiloxan mit einer Viskosität von 10.000 mPa.s bei 20° C, 200 Teilen dimethylhydrogensilyl-endgestopptem Polydimethylsiloxan mit einer Viskosität von 120 mPa.s bei 20° C, 450 Teilen des Quarzfeinstmehls von Beispiel 1, 18 Teilen anorganischem Farbpigment und 2 Teilen hydriertem Rizinusöl mit einem Schmelzpunkt von 85° C, wobei die Paste auf 95° C erwärmt und dann unter Rühren auf 25° C abgekühlt wurde.

Die Herstellung der Katalysatorpaste erfolgte durch Vermischen in einem Kneter von 526 Teilen vinylendgestopptem Polydimethylsiloxan mit einer Viskosität von 5.000 mPa.s bei 20° C, 470 Teilen des Quarzfeinstmehls von Beispiel 1, 1,8 Teilen Titandioxid, 2 Teilen hydriertem Rizinusöl mit einem Schmelzpunkt von 85° C, wobei die Paste auf 95° C erwärmt und dann unter Rühren auf 25° C abgekühlt wurde, und 0,2 Teilen des Platinsiloxan-Komplexes aus Beispiel 1.

Beispiel 4 (Vergleichsversuch)

In einem Kneter wurde die Basispaste hergestellt durch Vermischen von 245 Teilen vinylendgestopptem Polydimethylsiloxan mit einer Viskosität von 10.000 mPa.s bei 20° C, 160 Teilen dimethylhydrogensilyl-endgestopptem Polydimethylsiloxan mit einer Viskosität von 120 mPa.s bei 20° C, 520 Teilen des Quarzfeinstmehls aus Beispiel 1, 70 Teilen der gefällten Kieselsäure aus Beispiel 2 und 5 Teilen anorganischem Farbpigment.

Die Katalysatorpaste wurde in einem Kneter hergestellt durch Vermischen von 408 Teilen vinylendgestopptem Polydimethylsiloxan mit einer Viskosität von 5.000 mPa.s bei 20° C, 520 Teilen des Quarzfeinstmehls von Beispiel 1, 20 Teilen der gefällten Kieselsäure von Beispiel 2, 1,8 Teilen Titandioxid und 0,2 Teilen des Platin-siloxan-Komplexes von Beispiel 1.

Beispiel 5 (erfindungsgemäß)

Die Basispaste wurde hergestellt durch Vermischen in einem Kneter von 250 Teilen vinylendgestopptem Polydimethylsiloxan mit einer Viskosität von 10.000 mPa.s bei 20° C, 160 Teilen dimethylhydrogensilyl-endgestopptem Polydimethylsiloxan mit einer Viskosität von 120 mPa.s bei 20° C, 570 Teilen des Quarzfeinstmehls von Beispiel 1, 5 Teilen anorganischem Farbpigment und 15 Teilen hydriertem Rizinusöl mit einem Schmelzpunkt von 85° C, wobei die Paste auf 95° C erwärmt und dann unter Rühren auf 25° C abgekühlt wurde.

Die Herstellung der Katalysatorpaste erfolgte durch Vermischen in einem Kneter von 408 Teilen vinylendgestopptem Polydimethylsiloxan mit einer Viskosität von 5.000 mPa.s bei 20° C, 575 Teilen des Quarzfeinstmehls von Beispiel 1, 18 Teilen Titandioxid, 15 Teilen hydriertem Rizinusöl mit einem Schmelzpunkt von 85° C, wobei die Paste auf 95° C erwärmt und dann unter Rühren auf 25° C abgekühlt wurde, und 0,2 Teile des Platinsiloxan-Komplexes von Beispiel 1.

4

Alle Pasten wurden nach der Herstellung in 130 ml fassende Tuben mit einer Austrittsöffnung von 6 mm Durchmesser gefüllt und 24 Stunden bei 23° C gelagert. Danach wurde auf einem Anmischblock ein 10 cm langer Pastenstrang ausgedrückt. Hierbei wurde beurteilt, ob ein geringer oder großer Kraftaufwand notwendig ist, die Paste aus der Tube zu drücken, und zum anderen, ob der Pastenstrang eine Minute nach dem Ausdrücken noch standfest oder schon verlaufen ist.

Für die Viskositätsmessung stand ein Rotationsviskosimeter der Fa. Haake zur Verfügung und zwar die Platte-Kegel-Einrichtung PK 100 mit 3°-Kegel, Meßkopf M 500 und der Schreiber RV 100 mit entsprechenden Prüfprotokollen (Bestellnummer 222-0068). Auf diesen Prüfprotokollen wurden folgende Angaben eingetragen:

| | |
|---|---|
| Temperatur | 23°C |
| Rotovisko | RV 100 |
| System | M 500 |
| Meßeinrichtung | I 3° |
| A | 83,5 |
| M | 8,73 |
| Programmzeit $t_1$ | 0 |
| Programmzeit $t_2$ | 1 |
| Programmzeit $t_3$ | 0 |
| Schubspannung $S_\gamma$ | 10 % $\gamma$ (Beispiel 1-3) 20 % $\tau$ (Beispiel 4,5) |
| Geschwindigkeitsgefälle $S_D$ | 1 % D |

Die unten in Tabelle 1 angegebenen Viskositätswerte wurden aus den aufgezeichneten Viskositätskurven bei Punkt 0,2 und Punkt 1,0 des Geschwindigkeitsgefälles (x-Achse) errechnet.

Tabelle 1

| Beispiel | 1 (Vergleich) | | 2 (Vergleich) | | 3 (erfind.gem.) | | 4 (Vergleich) | | 5 (erfind.gem.) | |
|---|---|---|---|---|---|---|---|---|---|---|
| Paste | Basis-paste | Kataly-sator-paste | Basis-paste | Kataly-sator-paste | Basis-paste | Kataly-sator-paste | Basis-paste | Kata-lysa-tor-paste | Basis-paste | Kataly-sator-paste |
| Quarz + Farbstoff | 45 % | | 40 % | | 47 % | | 52,5 % | | 57,5 % | |
| Kieselsäure | – | | 5 % | | – | | 7 % | | – | |
| Hydr. Rizinusöl | – | | – | | 0,2 % | | – | | 1,5 % | |
| Kraftaufwand beim Tubenausdrücken | gering | gering | gering | gering | gering | gering | erhöht | erhöht | leicht erhöht | leicht erhöht |
| Standfestigkeit des Stranges | keine | keine | mäßig | mäßig | gut | gut | mäßig | mäßig | sehr gut | sehr gut |
| Visksoltät bei | | | | | | | | | | |
| Pt. 0,2 | 12 Pa.s | 12 Pa.s | 39 Pa.s | 39 Pa.s | 60 Pa.s | 57 Pa.s | 195 Pa.s | 186 Pa.s | 391 Pa.s | 418 Pa.s |
| Pt. 1,0 | 12 Pa.s | 12 Pa.s | 26 Pa.s | 27 Pa.s | 26 Pa.s | 25 Pa.s | 100 Pa.s | 92 Pa.s | 127 Pa.s | 135 Pa.s |
| Pt. 0,2 | 12 Pa.s | 12 Pa.s | 30 Pa.s | 30 Pa.s | 57 Pa.s | 55 Pa.s | 114 Pa.s | 102 Pa.s | 364 Pa.s | 382 Pa.s |

**Ansprüche**

1.  Additionsvernetzende Massen auf Polysiloxanbasis, enthaltend
     (a) ein Organopolysiloxan mit zwei oder mehreren Vinylgruppen im Molekül,
     (b) ein Organohydrogenpolysiloxan als Vernetzer,
     (c) einen Katalysator zur Beschleunigung der Additionsreaktion,

(d) hydrophobe Füllstoffe, sowie gegebenenfalls

(e) Farbstoffe/Pigmente, dadurch gekennzeichnet daß sie zusätztlich hydriertes Rizinusöl eines Schmelzbereichs von 80 bis 95° C enthalten.

2. Massen nach Anspruch 1, dadurch gekennzeichnet, daß sie 0,1 bis 3 Gew.-% bezogen auf Gesamtpaste an hydriertem Rizinusöl enthalten.

3. Massen nach Anspruch 1 oder 2, dadurch gekennzeichnet, daß das hydrierte Rizinusöl einen Schmelzbereich von 82 bis 90° C besitzt.

4. Verwendung von Massen gemäß Anspruch 1 bis 3 als Abformmassen.

5. Verwendung gemäß Anspruch 4 für Zahn-, Schleimhaut- und Modellabformungen.

## Claims

1. Materials which crosslink by addition and are based on polysiloxane, containing
   (a) an organopolysiloxane with two or more vinyl groups in the molecule,
   (b) an organo-hydridopolysiloxane, as a crosslinking agent,
   (c) a catalyst for accelerating the addition reaction,
   (d) hydrophobic fillers and, if appropriate,
   (e) dyestuffs/pigments, characterized in that they additionally contain hydrogenated castor oil with a melting range from 80 to 95° C.

2. Materials according to Claim 1, characterized in that they contain 0.1 to 3% by weight, based on the total paste, of hydrogenated castor oil.

3. Materials according to Claim 1 or 2, characterized in that the hydrogenated castor oil has a melting range from 82 to 90° C.

4. Use of materials according to Claims 1 to 3 as impression materials.

5. Use according to Claim 4 for impressions of teeth, mucous membranes and models.

## Revendications

1. Compositions à base de polysiloxanes, se réticulant par addition, contenant
   (a) un organopolysiloxane portant deux ou plus de deux groupes vinyle dans sa molécule,
   (b) un organohydrogénopolysiloxane comme agent de réticulation,
   (c) un catalyseur servant à accélérer la réaction d'addition,
   (d) des charges hydrophobes, ainsi que, le cas échéant,
   (e) des colorants/pigments, caractérisées en ce qu'elles contiennent en outre de l'huile de ricin hydrogénée fondant dans la plage de 80 à 95° C.

2. Compositions suivant la revendication 1, caractérisées en ce qu'elles contiennent 0,1 à 3% en poids d'huile de ricin hydrogénée, par rapport à la quantité totale de pâte.

3. Compositions suivant la revendication 1 ou 2, caractérisées en ce que l'huile de ricin hydrogénée possède une plage de fusion de 82 à 90° C.

4. Utilisation de compositions suivant les revendications 1 à 3 comme compositions à modeler.

5. Utilisation suivant la revendication 4 pour des modelages de dents, de muqueuses et de modèles.